# EUROPEAN PATENT SPECIFICATION

(11) **EP 2 960 862 B1**
(45) Date of publication and mention of the grant of the patent: **22.03.2017**
(21) Application number: 14002150.2
(22) Date of filing: 24.06.2014
(51) Int. Cl.: G06T 7/20, A61B 5/024

(54) **A method for stabilizing vital sign measurements using parametric facial appearance models via remote sensors**
Verfahren zur Stabilisierung von Lebenszeichenmessungen unter Verwendung parametrischer Physiognomiemodelle über Fernsensoren
Procédé de stabilisation des mesures de signes vitaux utilisant des modèles d'apparence faciale paramétrique via des capteurs à distance

(43) Date of publication of application: 30.12.2015
(73) Proprietor: Vicarious Perception Technologies B.V., 1015 AH Amsterdam (NL)
(72) Inventor: Tasli, H. Emrah, 1015 AH Amsterdam (NL); Gudi, Amogh, 1015 AH Amsterdam (NL); Ivan, Paul, 1015 AH Amsterdam (NL); Uyl, Marten den, 1015 AH Amsterdam (NL)
(74) Representative: Karaahmet, Erdogan

(56) References cited:
- US-A1- 2004 039 273
- DRAGOS DATCU ET AL: "Noncontact automatic heart rate analysis in visible spectrum by specific face regions", COMPUTER SYSTEMS AND TECHNOLOGIES, ACM, 2 PENN PLAZA, SUITE 701 NEW YORK NY 10121-0701 USA, 28 June 2013 (2013-06-28), pages 120-127, XP058034178, DOI: 10.1145/2516775.2516805 ISBN: 978-1-4503-2021-4

## Description

### Related Field of the Invention

This invention discloses a remote photo-plethysmography measurement system where human skin color variations are analyzed for observing human vital signs by using a non-invasive remote sensor.

The present invention further covers the areas of measuring the temporal changes in vital signs for monitoring and analyzing the physical and psychological state of the subject under observation.

Moreover, a facial region of interest definition is disclosed with an Active Appearance Model (AAM) generation for tracking the changes on the face and facial expressions in a manner to stabilize the signal acquisition step and obtain a noise free skin color variation.

### Background of the Invention (Prior Art)

Measurement and analysis of vital signs of a human attains a vital role in monitoring human physical and/or psychological health conditions. Such measurements are foreseen to be useful in the sense that such methods could make longer time predictive analysis possible and hence making the precautionary action to be a part of the treatment. Moreover, such methods could be used in the cases where invasive instruments for vital sign analysis are difficult or even impossible for various reasons, i.e. attachment/detachment of a sensor to a patient under intensive care could violate the sustained hygiene environment and requirements.

The conventional methods of vital sign measurements (including but not limited to heart rate, heart rate variability, respiratory rate, etc.) which utilize attached body sensors requiring an installation that could violate the hygienic conditions and/or cause a discomfort to the human in need of assistance. Therefore, techniques for preventing such difficulties have been widely investigated.

Methods utilizing visible light spectrum where no active lighting is applied or required have been presented in order to overcome the aforementioned difficulties and enable systems for obtaining remote monitoring of subjects for various physiological and psychological measurements.

Contactless methods are preferred due to their advantages compared to conventional methods. Such methods, previously defined in [1], [2] utilize the small variations of the skin color reflectance under visible light.

Remote photo-plethysmography (RPPG) measures the small deviations in color values in order to extract an amplified signal where the periodic heart and/or respiratory rate signal is detectable. The underlying principle is that blood absorbs light more than its neighborhood; hence, changes in blood volume affects the reflectance properties accordingly. The mentioned color variations are observed in relation to the oxygen levels of the hemoglobin and hence could reliably be used as an indicator for the periodic heart rate signal. By capturing the color information of the skin with a proper sampling rate, these variations could be recorded and analyzed. However, the process of remotely measuring skin color variations for the purpose of vital sign measurement, requires that the measured skin locations are either stable, or could be accurately tracked. Since the minor changes in color are essential for accurate vital sign measurement, even the smallest movements could cause a major degradation in the final performance.

It has been previously shown that it is possible to estimate vital signs remotely by using a passive, non-invasive RGB camera without active sensors. The plethysmographic measurements are realized using the skin color variations especially from the facial areas where the skin is usually not covered. However, the process of remotely measuring skin color variations for the purpose of vital sign measurement, requires that the measured skin locations are either stable, or could be accurately tracked. Since the minor changes in color are essential for accurate vital sign measurement, possible alignment issues might cause a major degradation in the final performance of the method.

In a prior study [1], where a method for remote photo-plethysmography measurement is explained, the said alignment issue is addressed using a passive detection mechanism as such the vital sign information is extracted from a fixed region of the face. The selection of the said fixed region on the face is experimented with different sizes and locations. The observed signal to noise levels under different region selection vary in accordance with the size and homogeneity of the selected region. It is therefore proposed to use averaged signal from the said fixed regions in a way to maximize the intra-region similarity and increase the signal to noise ratio. However, the proposed method could suffer from the case where the person moves his head such that the selected fixed regions would no longer correspond to an intended skin surface and no accurate signal acquisition would be possible.

In the other similar study [2], [3] a face detection method is proposed to overcome the said alignment issue in order to stabilize the captured color information. However, using a face detection method could not totally normalize the misalignment and hence an advanced mechanism is necessary. The prior study [8] proposes active appearance model to obtain 66 facial keypoints for evaluating different region of interests (ROI) for signal acquisition.

WO 2009/040711 A3, WO 2012/143842 A3, WO 2013/128345 A1, WO 2012/020365 A1, US 2013/0184517 A1 and WO 2011/060220 A1 can be regarded as examples for the above disclosed prior art.

Also, the below documents can be regarded as relevant prior art considering the present invention. These documents are reviewed in detail and substantial differences of the present invention are explained below.

### US 2014/0088433 A1 :

This invention is related to a device and a method for extracting physiological information from remotely detected electromagnetic radiation emitted or reflected by a subject. The method uses the following steps;
- A data stream derivable from electromagnetic radiation emitted or reflected by a subject is received,
- The data stream comprises a sequence of signal samples indicative of desired subject motion and of disturbing motion,
- The signal samples represent at least one region of interest exhibiting an at least partially periodic indicative motion pattern attributable to at least one physiological parameter, and a non-indicative motion region,
- The sequence of signal samples is processed, comprising deriving a sequence of derivative motion compensated samples at least partially compensated for undesired overall motion,
- Detecting an evaluation parameter representative of motion compensation accuracy,
- And deriving at least one characteristic signal at least partially indicative of the at least partially periodic indicative motion pattern from the sequence of motion compensated samples, wherein deriving the characteristic signal is performed depending on the detected evaluation parameter.

As the detection of the electromagnetic radiation emitted or reflected by a subject is different from the detection of the visible light, there are substantial differences between this document and the present invention such as;
- a processing unit configured for processing the sequence of signal samples is used in US 2014/0088433 A1 while a sequence of signal samples are processed to generate a facial model over which the temporal color variations are observed to measure the vital signs in the present invention and it is intended to extract facial color signal accurately in different facial states so that a robust signal acquisition, namely signal stabilization can be provided.
- a motion compensation assessment module configured for detecting an evaluation parameter representative of motion compensation accuracy is used in US 2014/0088433 A1 while the facial landmarks in the video are detected independently in the present invention which results in having no need for motion compensation.
- an analyzer configured for determining temporal variations in the characteristic signal is used in US 2014/0088433 A1 wherein the temporal variations are being representative of at least one vital signal. Unlike US 2014/0088433 A1, different states of face are not compared in the present invention but temporal variations in the color signal are analyzed for vital sign measurement.

### WO 2013/186696 A1 :

This invention discloses a system which is capable of measuring the vital signs such as a respiratory rate or a heart rate by using an imaging unit (2) for obtaining video data of the subject, a marker (10, 20, 60, 61) directly or indirectly attached to a body of the subject, wherein the marker comprises a graphical pattern (11,21), an image processing unit (3) for detecting said marker in said video data, and an analysis unit (4) adapted to extract a vital sign parameter related to the vital sign of the subject from said video data and to determine the vital sign from said vital sign parameter. The system also uses a software to execute the above mentioned properties.

However, WO 2013/186696 A1 focuses on a scenario where the patient is lying in a bed and monitored using a remote camera using the initial markers to find the region of interest.

Unlike WO 2013/186696 A1 the present invention discloses a method that would aim for a general scenario where the subject is no longer limited to a bed or to a lying positions. Therefore the present invention provides a generic solution with the improved algorithmic pipeline for stabilizing the vital signals using active appearance model obtained from a face where the a marker is not required for region of interest estimation.

### WO 2013/156908 A1 :

This invention discloses a processor and a corresponding processing method for obtaining vital sign information of a living being from an image signal generated from spatiotemporal variations of received light registered in at least one wavelength interval.

WO 2013/156908 A1 intends to increase the quality of "vital sign" signal using a control unit by supplying a feedback to the registration unit (camera). However, the method of how this should be realized is not detailed in the description.

The present invention mainly differs from this document in the sense that no control unit is used to update the parameters of the camera. Instead, the present invention models the image (face) parametrically to overcome these post-checking issues.

WO 2013/156908 A1 further vaguely mentions about region selection: *"Said ROI(s) may be selected either automatically, or manually, and not for the entire image. For this purpose the proposed device may further comprise a election unit for automatically selecting said region of interest or allowing a manual election of said region of interest."*

In the present invention on the other hand, the way how signal stabilization is achieved is explained in detail with required references to the literature.

### CN 102499664 A:

This invention discloses a video-image-based method for detecting a non-contact vital sign. The method comprises the following steps of: acquiring video images continuously according to a fixed frame frequency, detecting a region of interest (ROI) region automatically, separating a vital sign signal from a multichannel signal separated from the ROI region, extracting the frequency of the vital sign signal and converting the frequency into the vital sign to acquire a detection result. The invention also discloses a video-imagebased system for detecting the non-contact vital sign. The method and the system have the advantages of quickness, real time, capacity of realizing continuous monitoring, high robustness, low cost, wide application range and the like.

As a conclusion, the invention offers a vital sign detection method using the main harmonic frequency estimation of the vital sign. The signal frequency is estimated using FFT and ICA methods for robustness. Such methods for obtaining vital sign measurements have been previously proposed in international conferences.

On the other hand, the present invention discloses a novel method using the time signal and obtaining noise free signal using the parametric facial analysis and double detrending techniques.

The paragraphs below are Chinese to English translations of the corresponding parts of the description.

*"Detection method wherein said frequency of said vital signs including heart rate and respiratory frequency signal frequency of the signal i;. The vital signs including heart rate, respiration, the detection method according to claim 2, which characterized in that the vital signs of the heart rate, the RCH target area of the skin area is measured; the vital signs of breathing, the subject under test ROI chest region or abdominal position, as described in claim..."*

*"The 0 detection method wherein said periodic signal frequency measurement method is a multi-ffi picking relevant law, said source signal points for multiple correlation operation into line frequency analysis, the spectrum of the peak power point of the vital signs. "*

Dragos Datcu et. al. "Noncontact Automatic Heart Rate Analysis in Visible Spectrum by Specific Face Regions" International Conference on Computer Systems and Technologies. 2013

This paper presents a method to compute heart rate and uses Active Appearance Models (AAM) for extraction of the 66 keypoints on the face. As soon as the shape is extracted, the methods described in [9], [12], [13] (citations are as referred in the original text are applied to compute the heart rate signal. The average RGB signal is first detrended, then normalized and the ICA (independent Component Analysis) applied. Finally, the FFT (Fast Fourier Transform) is applied to find the periodic signal, namely the heart rate.

### Brief Description and Aims of the Invention

It is the purpose of the present invention to provide a method for measuring vital signs that allow free head movement with the proposed facial modelling.

A method according to the invention is defined in claim 1. The active appearance models technique is used to define the facial parameters in order to locate the facial landmarks and generate the normalized appearance image over which the vital sign measurement could be done. With the said normalization the locational and color changes due to facial expression changes, muscle and head movements could be compensated.

The lack of accuracy in prior art for said region definition and stability is resolved with the disclosed shape and AAM generation.

Hence, a system and method for vital sign measurement using an off-the-shelf video recording device with sufficient computational power is presented.

The system further comprises a facial modeling capability with an adaptive region of interest selection wherein the signal fluctuation is limited.

The application of the system under varying conditions where the subject is sleeping, sitting or travelling is possible as long as the main orientation of the face is directed towards the video recording device such that the visible facial region is maximized for an accurate active appearance modelling.

The present disclosure also relates to a method of measuring humanly vital signs, comprising of a video recording from a digital medium of human face of at least one human subject as the subject is limitedly free to act and move during the measurement; using the digital medium for automatically detecting and describing each of a plurality of selected facial landmarks by conducting an artificial intelligence method; automatically detecting the landmarks and stabilizing on the video sequence; wherein the said adaptive region candidates on the facial locations are replaced with shape and appearance based visual adaptations; conducting a mathematical evaluation on the selected single or multiple regions of interest based upon a selected single or multiple color channels recorded within a predetermined time period.

This allows obtaining a single or multiple shape adaptive region of interest definition for controlling the signal acquisition step so that the PPG related information is preserved wherein the facial expressions and muscle movements are considered.

The system can further be applied on a plurality of subjects in the same scene given that the face is directed towards the camera such that the visible facial region is maximized for an adequate active appearance modelling. The plurality of the subjects is determined and limited by the processing power of the computer device, lighting conditions in the environment, distance and resolution of the recording device.

According to the invention, the captured signal is further processed for obtaining the relevant periodic components. The average signal generated by the active appearance model is processed such that the effects of very low frequency color changes due to a change in light intensity or appearance model could be removed. In the proposed invention the detrended signal is subtracted from the raw signal for obtaining a smoothed signal where the high frequency fluctuations is mostly removed. A second detrending operation is performed to obtain the smooth periodic PPG signal over which the average heart rate and interbeat interval measurements are confidently conducted.

The system is further capable of detecting the presence of a person and automatically deactivating the vital sign measurement system for energy and computational efficiency purposes.

The proposed invention provides a robust and widely applicable method where a conventional camera is required to record the a scene where one or multiple human subjects are present under adequate lighting conditions and good camera angle such that the active appearance model is well fitted to the actual image so that the region of interest is accurately captured and tracked during the footage until sufficient number of samples are obtained. The invention could well be applied for cases where remote monitoring is necessary and required. The proposed active appearance model satisfies a robust color acquisition for further vital sign measurement analysis.

### Definition of the Figures

In order to explain the present invention in more detail, the following figures have been prepared and attached to the description. The list and the definition of the figures are given below.
**FIGURE 1** shows the 491 landmark points of a neutral face from the frontal and lateral view
**FIGURE 2** illustrates the functional pipeline of the process

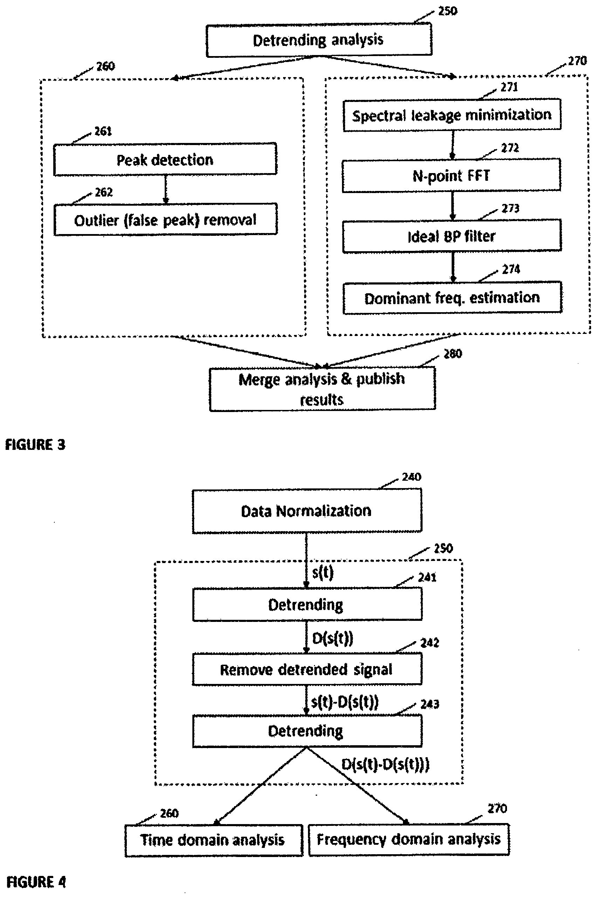

**FIGURE 3** illustrates the details of frequency and time analysis
**FIGURE 4** illustrates the details of detrending analysis
**FIGURE 5** Raw signal of a single color channel, s(t)
**FIGURE 6** Detrended raw signal of a single color channel, D(s(t))
**FIGURE 7** Noise removal by subtracting detrended signal from raw signal, s(t)-D(s(t))
**FIGURE 8** Detrending the noise removed signal (Second detrending), D(s(t)-D(s(t)))
**FIGURE 9** Time domain analysis
**FIGURE 10** Frequency domain analysis
**FIGURE 11** is the flow chart of pre-heating, continuous heating and implementation of the internal heating system

### Definition of the Elements (Features/ Components/ Parts) on the Figures

The definition of the features/components/parts that are covered in the figures that are prepared in order to explain the present invention better are separately numbered and given below.
- 100.: System start
- 110.: Frame capture
- 200.: Face detection [5]
- 210.: Active shape and appearance based facial modelling [7]
- 220.: System search for the same region on the current frame
- 230.: Signal buffer
- 240.: Data normalization
- 241.: First detrending
- 242.: Removal of the detrended signal
- 243.: Second detrending
- 250.: Detrending analysis
- 260.: Time domain analysis
- 261.: Peak detection
- 262.: Outlier removal
- 270.: Frequency domain analysis
- 271.: Spectral leakage minimization
- 272.: Realization of frequency domain transformation
- 273.: Ideal band-pass frequency filtration
- 274.: Domain frequency estimation
- 280.: Final step
- 300.: Analysis reset

### Detailed Description of The Invention

The presented disclosure relates to a novel and instrumental method for estimating humanly vital signs using a remote non-invasive photo-plethysmography (PPG) measurement system. Particularly, the invention focuses and elaborates on the facial modelling based signal acquisition step where the deficiency of the prior art is mainly observed. More particularly, using the active appearance model, movement of the head, moreover the facial landmarks are tracked such that the captured facial color values are replaced with the parametric active appearance model vector. This produces increased accuracy and stability compared to the prior art where a fixed region [1] or face detection based region of interest [2], [3] is utilized.

The present invention, in a preferred embodiment, further relates to the parametric modelling of the face using an annotated training set where the facial landmark points are identified by the human annotations for the purpose of developing an artificial intelligence method for identifying the landmark points of an unknown face.

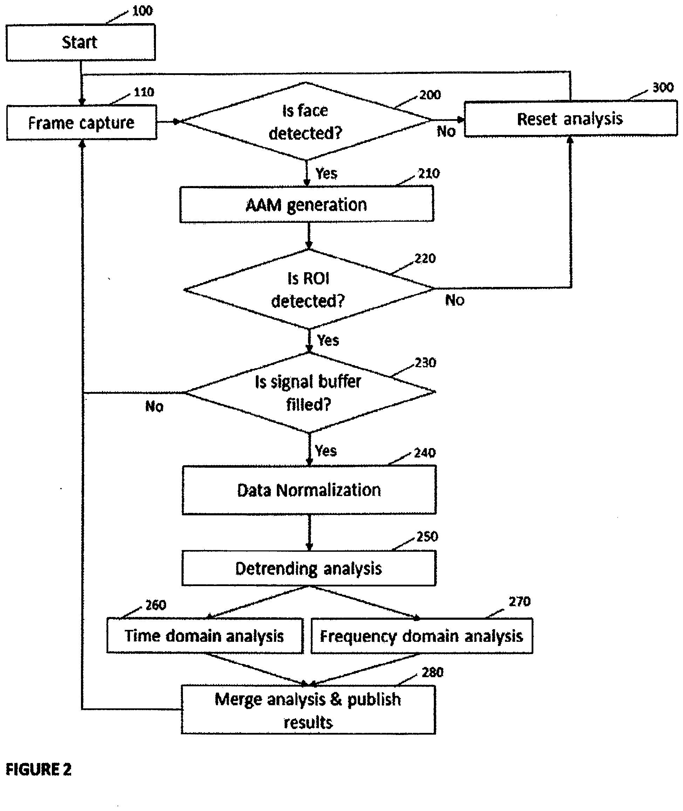

**Figure 2** illustrates the functional pipeline of the process. When the system is started **100**, system grabs a new frame **110** either from a previously recorded video or an online monitoring system that captures images in a real time setup. The new frame capture is also initiated at different steps of the pipeline; at the final step **280**, after an analysis reset **300** or if the signal buffer **230** is incomplete.

The widely accepted Viola & Jones face detection method [5] is performed **200** before the actual facial modelling **210**. If there is no face detected in the frame, the whole analysis is reset **300** and terminated.

If face is detected, it is further analyzed for determining the underlying landmark locations. The active appearance modeling [6] framework is utilized at this step for generating the active shape and appearance [7] based facial modelling **210**.

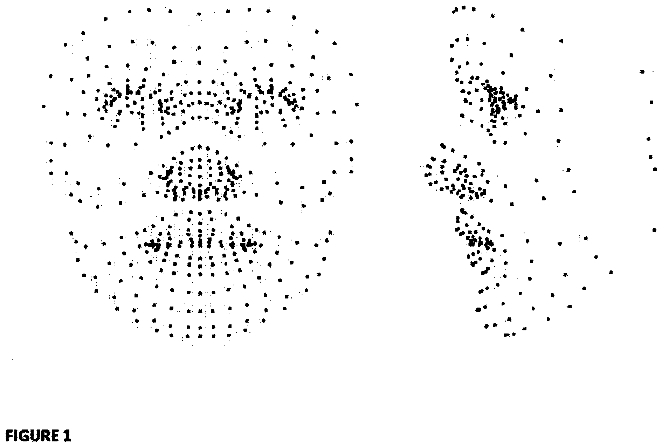

The 491 facial landmark points generated by active appearance modelling as shown in **Figure 1** provide the model of the face for the given head pose and facial expression. The detected 3D facial landmark points are used to parameterize the shape and appearance of the face in the current frame. The detection of such landmark points enable temporal stabilization of the facial regions and hence increasing the robustness of the vital sign measurement system under scenarios where the head of the person moves.

In the currently captured frame, if the region of interest (ROI) definition is available from the previously captured frame, system conducts a search **220** for the same region on the current frame.

If the previous ROI is not available, the analysis is reset **300** and a new frame is captured **110**.

Possible reasons for such a case could be that the region of interest on the visible face is no more available due to occlusion or changed head pose. If the previous ROI is available, but signal buffer **230** is not filled properly, new frame capture module is started and a new frame is captured **110** without resetting the parameters. This is because the system requires a predefined number of frames before performing an accurate vital sign analysis.

In the data normalization step **240**, the mean of the color signal in the selected time window is subtracted from itself. Hence the DC part of the signal is removed and only the periodic signal is obtained. This signal is further normalized to (-1, 1) for analysis.

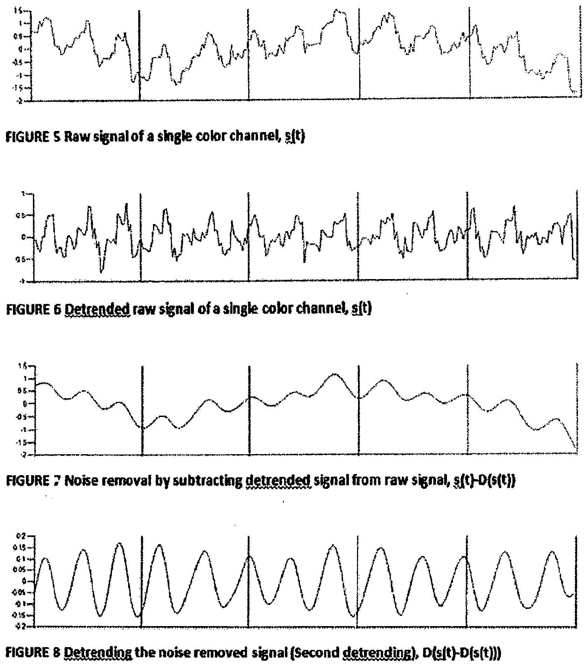

**Figure 5** shows a single color channel of the normalized raw signal in a selected window period. In the detrending analysis **250** following the normalization, aperiodic trend in the signal is removed as shown in **Figure 6**. The color signal is directly acquired from the adaptive facial regions or alternatively, the parametric facial appearance model could be used as the raw signal in the system.

Measuring vital signals has been previously addressed in prior art [1], [2]. The conventional way of running the analysis is executed by gathering and averaging the color data from a fixed but wide face region and the hardly visible changes in the color values are amplified in order to detect the primary frequency component which in fact corresponds to the heart rate. Moreover, even lower frequency respiratory rate could also be extracted using the lower frequency components.

With the present invention it is possible to reach high accuracy measurements with the generated active appearance model parameters. Instead of using the raw color data from the facial regions, the appearance model is utilized for obtaining the modelled color of the facial region of interest.

The time **260** and frequency 270 domain analysis extract the periodic signal information from the detrended signal values. The two independent analysis are merged at the final step **280** for quality analysis. At the merging step, the confidence from the time and frequency domain measurements are integrated. Time domain confidence is estimated using the magnitude and variation of the heart rate peaks. Strong peaks with minimal variation (in a window of ~8 seconds) encourage higher confidence. Frequency domain confidence is dependent on the peak frequency magnitude with respect to the remaining frequency components. Strong dominant peak indicate higher confidence.

If the two analysis do not agree, a confidence voting between the time and frequency domain estimation is used before publishing the final analysis results. The analysis reveals important vital signs including but not limited to heart rate, respiratory rate and heart rate variability. These signs could be useful for monitoring and exploring the physical and psychological state of the human subject. Upon the end of the explained analysis steps, next frame capture **110** is initiated.

The presented invention particularly relates to a novel detrending analysis where the normalized signal s(t), shown in **Figure 5**, is initially subjected to first detrending **241** to generate the detrended signal **D(s(t))** as shown in **Figure 6**. Prior methods run the time and frequency analysis on the data extracted from this signal. Proposed invention further exploits this step by subtracting **D(s(t))** from **s(t)** to remove the detrended signal (the high frequency components) **242**.

The filtered signal **s(t)-D(s(t))** as shown in **Figure 7** has a strong periodic component where the noise is strongly removed. A second detrending **243** step provides the final signal **D(s(t)-D(s(t)))** with a strong dominant frequency that can be directly used in the time and frequency analysis. The final signal is shown in **Figure 8**.

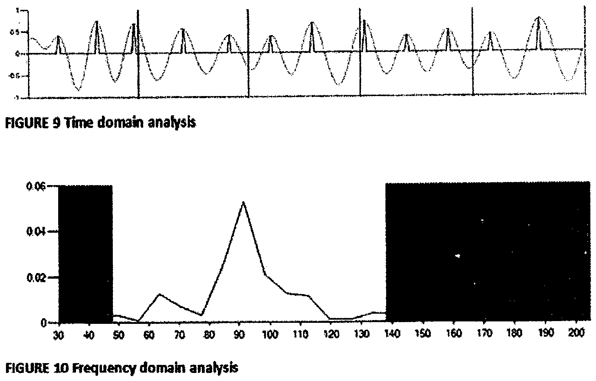

The periodic component of the signal is analyzed in two different steps. In the time domain analysis **260**, peak detection **261** is applied to the detrended time signal. The estimated peak intervals as shown in **Figure 10** are used as inter-beat-intervals (IBI) of the heart rate signal. Heart rate and respiratory rate are strongly correlated and the temporal variability of these signals are physically limited. Therefore, an additional outlier removal step **262** is utilized where possible wrong peak detections are removed for stability and accuracy purposes.

In a parallel setup, the detrended color signal goes through a frequency domain analysis **270**. In this analysis, the time domain signal is multiplied with a window function for minimizing the spectral leakage **271** on the frequency domain conversion. Hamming and Hann are popular window filters that could be applied at this step.

The frequency domain transformation is realized **272** with the discrete N-point FFT method. Since the frequency of vital signs could be limited between specific values, it is wise to ignore some frequency components by using ideal band-pass frequency filter **273**. At the final step, the dominant peak of the frequency signal in the selected **274** band-pass filter is accepted as the frequency of the vital sign as shown in **Figure 9**. The gray areas correspond to our filtered frequencies.

The major novelties of the presented invention are in the data acquisition and signal processing steps. Firstly, the color values for the vital sign analysis are alternatively obtained using the generated appearance model as well as the actual color values from the adaptive facial regions. Moreover, the proposed detrending step effectively removes the noise component of the signal where more accurate periodic component extraction becomes possible. Moreover, proposed method makes it possible to estimate IBI changes in the heart rate, this is in contrast to the prior art where frequency based measurements could only track average heart rate in the selected time window.

### REFERENCES:

[1] W. Verkruysse, L. O. Svaasand, and J. S. Nelson; "Remote plethysmographic imaging using ambient light", Optics Express, December 2008
[2] M. Z. Poh, D. J. McDuff, and R. W. Picard; "Non-contact, automated cardiac pulse measurements using video imaging and blind source separation," Opt Express, May 7, 2010.
[3] M. Z. Poh, D. J. McDuff, and R. W. Picard; "Method and system for measurement of physiological parameters", US Patent 2011/0251493 A1
[4] G. J. Edwards, C. J Taylor, T. F. Cootes; "Interpreting face images using active appearance models". Proceedings IEEE International Conference on Automatic Face and Gesture Recognition 1998
[5] P. Viola and M. Jones; "Robust Real-time Object Detection", International Journal of Computer Vision 2001
[6] M. J. den Uyl, H. van Kuilenburg; "The FaceReader: Online facial expression recognition" International Conference on Methods and Techniques in Behavioural Research, 2005.
[7] H. van Kuilenburg, M. Wiering, M.J. den Uyl; "A Model Based Method for Automatic Facial Expression Recognition", European Conference on Machine Learning (ECML) 2005.
[8] D. Datcu, M. Cidota, S. Lukosch, L. Rothkrantz; "Noncontact Automatic Heart Rate Analysis in Visible Spectrum by Specific Face Regions"; International Conference on Computer Systems and Technologies, 2013.

## Claims

1. A method for estimating humanly vital signs by using a remote non-invasive photo-plethysmography measurement system and wherein an active appearance model is used to model a face and track facial landmark points such that captured facial color values are replaced with a parametric appearance model vector wherein the said method comprises the steps of;
• grabbing a new frame 110 either from a previously recorded video or an online monitoring system that captures images in a real time setup,
• performing a face detection method 200 before the actual facial modelling 210,
• resetting 300 the whole analysis if there is no face detected in the frame,
• Utilizing active shape and appearance based facial modelling 210,
• obtaining the model of the face for a given head pose and facial expression via facial landmark points generated by active appearance modelling,
• using the detected 3D facial landmark points to parameterize the shape and appearance of the face in the current frame,
• in the currently captured frame, if a region of interest (ROI) definition is available from the previously captured frame, conducting a search for the same region in the current frame,
• resetting (300) the analysis and capturing a new frame (110) if the region of interest is not available,
• in a first detrending step, subtracting from the raw color signal s(t), the mean of the color signal in a selected time window at a data normalization step (240) in order to remove the DC part of the signal, further normalizing the signal to (-1,1) for analysis, thus obtaining a detrended raw signal D(s(t)),
• removing noise by subtracting the detrended raw signal Ds(t) from the raw signal s(t) for obtaining a signal s(t) - D(s(t)), and in a second detrending step, detrending the obtained signal s(t) - D(s(t)) to remove the remaining aperiodic trend in the signal,
• extracting the periodic signal information from the twice detrended signal via a time 260 and frequency 270 domain analysis,
• merging the two independent analyses at a final step 280 for quality analysis by means of integrating the time and frequency domain measurements wherein,
a. the time domain confidence is estimated using the magnitude and variation of heart rate peaks, and
b. the frequency domain confidence is dependent on the peak frequency magnitude with respect to the remaining frequency components
• using a confidence voting between the time and frequency domain estimation before publishing the final analysis results if the two analyses do not agree,
• capturing the next frame (110) upon the end of the analysis steps.

2. The method of claim 1, **characterized in that** parametric modelling of the face is conducted using an annotated training set where the facial landmark points are identified by the human annotations for the purpose of developing an artificial intelligence method for identifying the landmark points of an unknown face.

3. The method of claim 1, **characterized in that** the new frame capture 110 is initiated at the final step 280, after an analysis reset 300 or if the signal buffer 230 is incomplete.

4. The method of claim 1, **characterized in that** the color signal is directly acquired from the facial region of interest or alternatively, the active appearance model parameters could be used as the raw signal in the system.

5. The method of claim 1, **characterized in that** the analysis reveals important vital signs including but not limited to heart rate, respiratory rate and heart rate variability.

6. The method of claim 1, **characterized in that** the signal s(t)-D(s(t)) has a strong periodic component where the noise is strongly removed.

7. The method of claim 1, **characterized in that** a second detrending 243 step provides the final signal D(s(t)-D(s(t))) with a strong dominant frequency that can be directly used in the time 260 and frequency 270 domain analysis.

8. The method of claim 1, **characterized in that** the periodic component of the final signal is analyzed in two different steps wherein;
• the time domain analysis 260 comprises the steps of;
∘ applying peak detection 261 to the detrended time signal,
∘ using the estimated peak intervals as inter-beat-intervals of the heart rate signal
∘ strongly correlating the heart and respiratory rate to physically limit the temporal variability of the estimated values,
∘ utilizing an additional outlier removal step (262) for removing possible wrong peak detections,
and
• the frequency domain analysis (270) comprises the steps of;
∘ multiplying the time domain signal with a window function for minimizing the spectral leakage (271) on the frequency domain conversion,
∘ realizing the frequency domain transformation (272) with the discrete N point FFT method,
∘ ignoring some frequency components by using and ideal band-pass frequency filter (273),
∘ accepting the dominant peak of the frequency signal in the selected (274) band-pass filter as the frequency of the vital sign at the final step.

9. The method of claim 1, **characterized in that** the color values for the vital sign analysis are alternatively obtained using the generated appearance model as well as the actual color values from the facial region of interest.

10. The method of claim 1, **characterized in that** the detrending step effectively removes the noise component of the signal.

11. The method of claim 1, **characterized in that** inter-beat-interval changes in the heart rate are estimated.

12. The method of claim 1, **characterized in that** it comprises a single or multiple region of interest definition for controlling the signal acquisition step so that the PPG related information is preserved wherein the facial expressions and muscle movements are considered.

13. The method of claim 1, **characterized in that** the facial muscle and head movements are compensated to allow free head movement as signal acquisition is conducted using the active appearance facial landmark tracking.

## Patentansprüche

1. Verfahren zur Abschätzung des menschlischen vitalen Funktionen durch Verwendung des von fernen nicht insavien Photo - Plethysmographie Messsystems, wobei man durch das Ersetzen des parametrischen Erscheinung-Modellvektors von den erfassten Gesichtsfarbwerten das Gesichtsmodell ausbildet, wobei man ein aktives Erscheinungsmodell verwendet, um die vitale Punkte des Gesichts zu verfolgen und wobei hier das oben genannte Vefahren die folgenden Schritte umfasst;
• Erhalten eines neuen Rahmens (110), egal ob er aus einem aufgezeichneten Video oder aus einem Online-Monitoring-System, das die Bilder in einem Echtzeitsystem erfasst, aufgenommen ist,
• Realisieren eines Gesichtserkennungsverfahren (200) vor der Modellierung (210) des wahren Gesichts
• Neueinstellung aller Analyse, wenn kein Gesicht im Rahmen festgestellt ist,
• Verwendung der aktiven Form und Erscheinung bezüglich der Modellierung (210) des Gesichts,
• Erhalten eines Modelles für Kopfhaltung und Gesichtsausdruck mittels markierten Punkte des Gesichts, die durch die Modelliesierung der aktiven Erscheinung ausgebildet sind,
• Verwenden der markierten drei dimensionalen Punkte, die für das Bilden der Parameter der Form und Erscheinung des Gesichts in dem letzten Rahmen festgestellt sind,
• Durchführen einer Suche für den gleichen Bereich in dem letzten erfasasenen Rahmen, wenn in dem letzten erfassenen Rahmen die Definition des vorigen Rahmens für interessierende Region (ROI) vorhanden ist,
• Neueinstellung aller Analyse (300) und Erfassen eines neuen Rahmen (110), wenn interessierende Region nicht vorhanden ist,
• Bei dem ersten Schritt für den verwaltetenTrend, das Subtrahieren des Mittelwerts des Farbsignals vom rohen Farbsignal s(t) in einem Zeitfenster, das bei einem Schritt für Datennormalisierung (240) ausgewählt ist, um den DC Abschnitt des Signals zu beseitigen,
• Normaliesierung des Signals zusätzlich für die Analyse (-1, 1) und damit das Erhalten des rohen Signals Ds(t), dessen Trend verwaltet ist.
• Beseitigen des Lärms durch Subtrahieren des rohen Signals Ds(t) vom rohen Signal s(t) dessen Trend verwaltet ist, um ein Signal s(t) - D(s(t)) zu erhalten,
• Entfernen des Trends vom s(t) - D(s(t)) Signal, um den nicht-periodischen Trend bei dem zweiten Schritt für den verwalteten Trend zu beseitigen,
• Beschaffen der Information über den periodischen Signal aus dem zweimal entfernten Signal durch die Dauer- (260), und Frequenzanalyse (270),
• Kombinieren der beiden unabhängigen Analysen bei dem letzten Schritt (280) für die Qualitätsanalyse durch das Integrieren der Messungen des Dauers und der Frequenz.
a. Die Vertrauensrate für die Zeitdomänen schätzt man durch die Verwendung der Größe und der Variation vom Spitzenpunkt des Herzschlags und
b. Die Vertrauensrate für die Frequenz ist abhängig von der Spitzenfrequenzgröße bezüglich der Frequenzkomponenten.
• Durchfüheren einer Vertrauensabstimmung zwischen den Schätzungen über Zeit-, und Frequenzbereiche vor dem Veröffentlichen der Analysenergebnisse, wenn die beiden Analysen verschieden voneinander seien,
• Erfassen des nächsten Rahmen (110) nach dem Beenden der Analyseschritten.

2. Verfahren nach Anspruch 1 **dadurch gekennzeichnet, dass** die parametrische Modellierung des Gesichts durch Verwenden eines detaillierten Trainingssatzes erfolgt, wobei hier die markierten Punkte des Gesichts durch die menschlichen detaillierten Angaben definiert sind, um die künstliche Intelligenz für die Definition der markierten Punkte von einem unbekannten Gesicht zu entwickeln.

3. Verfahren nach Anspruch 1 **dadurch gekennzeichnet, dass man** den neu erfassten Rahmen (110) nach einer Neueinstellung einer Analyse (300) oder im Falle der Nichtvervollständigung einer Signalpufferung (230) im letzten Schritt anfangen läßt.

4. Verfahren nach Anspruch 1 **dadurch gekennzeichnet, dass** man die Farbensignale aus den betreffenden Gesichtsbereichen direkt erhält oder alternativ dazu die Parametern der Modell für aktive Erscheinung als rohes Signal im System verwendet werden können.

5. Verfahren nach Anspruch 1 **dadurch gekennzeichnet, dass** die Analyse die wesentlichen vitalen Signalen zeigt, die die Herzfrequenz , die Atemfrequenz und die Herzfrequenz -Variabilität enthalten und dagegen mit diesen nicht beschränkt sind.

6. Verfahren nach Anspruch 1 **dadurch gekennzeichnet, dass** das s(t)-D(s(t)) Signal eine starke periodische Komponente aufweist und der Lärm hier stark beseitigt ist

7. Verfahren nach Anspruch 1 **dadurch gekennzeichnet, dass** der zweite Schritt für den verwalteten Trend bei der Analyse über Dauer- (260), und Frequenzbereich (270) das letzte Signal D(s(t))- D(s(t))) bietet, so dass es direkt mit einer starken dominanten Frequenz verwendet werden kann

8. Verfahren nach Anspruch 1 **dadurch gekennzeichnet, dass** die periodische Komponente des letzten Signals in verschiedenen Schritten analysieren wird und hier,
• Daueranalyse (260) umfasst die folgenden Schritte:
∘ Durchführen der Spitzenerkennung (261) für das verwaltete Zeitsignal,
∘ Verwenden die geschätzten Spitzbereichen als Zeitintervall zwischen Herzschlag Signalrate,
∘ Leistungsfähige Beziehung der Herzschlagrate und der Atemfrequenz, um die zeitliche Variabilität der geschätzten Werte physikalisch zu begrenzen,
∘ Anwendung eines Beseitigungsschritts (262) um die fehlerhafte Spitzenfestlegungen zu entfernem,
und
• Frequenzanalyse (270) umfasst die folgenden Schritte:
∘ Multiplikation des Zeitbereichssignals mit einer Fensterfunktion, um die spektrale Leckage bei Frequenzbereich-Transformation (271) zu minimisieren,
∘ Realisieren der Frequenzbereich-Transformation (272) durch die N Punkt FFT Methode,
∘ Ignorieren der einigen Frequenzkomponenten durch Verwenden eines idealen Frequenzbandpassfilters (273)
∘ Annehmen das Frequenzsignal in dem gewählten Bandpassfilter (274) als Häufigkeit der Lebenszeichen im letzten Schritt der dominanten Spitze.

9. Verfahren nach Anspruch 1 **dadurch gekennzeichnet, dass** durch Verwenden durch der wahren Farbwerte des Gesichtsbereiches ausgebildete Erscheinungsmodell die Farbwerte für die vitalen Symptom-Analyse in einer alternativen Form erhalten werden.

10. Verfahren nach Anspruch 1 **dadurch gekennzeichnet, dass** der verwaltete Trendschritt die Aussparungskomponente des Signals in effektiver Weise beseitigt.

11. Verfahren nach Anspruch 1 **dadurch gekennzeichnet, dass** man die Zeitintervallunterschiede zwischen den Herzschlagraten schätzen kann.

12. Verfahren nach Anspruch 1 **dadurch gekennzeichnet, dass** es die Definitionen der einen oder mehreren interessierenden Regionen umfasst, um die Signalerfassungsschritt für das Speichern der betreffenden Information über PG zu steuern und hier der Gesichtsausdruck und die Muskelbewegungen berücksichtigt werden.

13. Verfahren nach Anspruch 1 **dadurch gekennzeichnet, dass** aufgrund der Realisierung der Signalerfassung durch die Gesichtsmarkenverfolgung der aktiven Erscheinung von Bewegungen der Gesichtsmuskeln und des Kopfes die Ermöglichkeit der freien Kopfbewegung kompensiert wird.

## Revendications

1. Procédé pour estimer des signes vitaux humains en utilisant un système de mesure de pléthysmographie non invasive à distance et dans lequel un modèle d'apparence actif est utilisé pour modéliser un visage et suivre des repères faciaux de telle sorte que les valeurs de couleur faciale capturées sont remplacées par un vecteur modèle d'apparence paramétrique dans lequel ledit procédé comprend les étapes consistant à:
- Prendre un nouveau cadre (110) soit à partir d'une vidéo précédemment enregistrée, soit par un système de surveillance en ligne qui capture des images dans une configuration en temps reel,
- réaliser une méthode de détection de visage (200) avant la modélisation faciale réelle (210),
- Réinitialiser (300) l'analyse entière s'il n'y a pas de visage détecté dans le cadre,
- Utiliser la modélisation faciale baseé sur la forme active et l'apparence (210),
- Obtenir le modèle du visage pour une pose de tête donnée et une expression faciale via des repères faciaux générés par la modélisation d'apparence active.
- L'utilisation des repères faciaux 3D détectés pour paramétrer la forme et l'apparence du visage dans le cadre actuel,
- Dans le cadre actuellement capturé, si une définition de région d'intérêt (ROI) est disponible à partir du cadre précédemment capturé, effectuer une recherche pour la même région dans le cadre actuelle,
- Réinitialiser (300) l'analyse et la capture d'un nouveau cadre (110) si la région d'intérêt n'est pas disponible,
- dans une première étape d'élimination de tendances, soustraire du signal de couleur brute s (t) la moyenne du signal de couleur dans une fenêtre de temps sélectionnée à une étape de normalisation de données (240) afin d'éliminer DC partie du signal
- normaliser le signal à (-1, 1) pour l'analyse, obtenant ainsi un signal brut éliminé D (s(t)),
- supprimer le bruit en soustrayant le signal brut éliminé Ds(t) du signal brut s (t) pour obtenir un signal s(t) -D (s(t))
Et dans une seconde étape d'élimination de tendances, éliminer le signal obtenu s(t) -D (s(t)) pour supprimer la tendance apériodique restante dans le signal,
- extraire les informations de signal périodique à partir du signal éliminé deux fois par une analyse de domaine de temps (260) et de fréquence (270),
- Fusionner les deux analyses indépendantes lors d'une étape finale (280) pour l'analyse de qualité en intégrant les mesures de domaine de temps et de fréquence dans lesquelles,
a. la confiance dans le domaine temporel est estimée en utilisant l'amplitude et la variation des crêtes de fréquence cardiaque et
b. la confiance du domaine fréquentiel dépend de l'amplitude de fréquence de crête par rapport aux composantes de fréquence restantes
- utiliser un vote de confiance entre l'estimation du domaine temporel et fréquentiel avant de publier les résultats de l'analyse finale si deux analyses ne concordent pas,
- capturer le cadre suivant (110) à la fin des étapes d'analyse.

2. Procédé selon la revendication 1, **caractérisé en ce que** la modélisation paramétrique de la face est réalisée en utilisant un ensemble d'apprentissage annoté où les points de repères faciaux sont identifiés par les annotations humaines dans le but de développer une méthode d'intelligence artificielle pour identifier les points de repères d'un visage inconnu.

3. Procédé selon la revendication 1, **caractérisé en ce que** la nouvelle capture de cadre (110) est initiée à l'étape finale (280) après une réinitialisation d'analyse (300) ou si le tampon de signal (230) est incomplet.

4. Procédé selon la revendication 1, **caractérisé en ce que** le signal de couleur est directement acquis à partir des régions faciales ou bien alternativement le modèle d'apparence peut être utilisé comme signal brut dans le système.

5. Procédé selon la revendication 1, **caractérisé en ce que** l'analyse révèle d'importants signes vitaux incluant sans s'y limiter, la fréquence cardiaque, la fréquence respiratoire et la variabilité de la fréquence cardiaque.

6. Procédé selon la revendication 1, **caractérisé en ce que** le signal s (t) -D (s (t)) présente une composante périodique forte où le bruit est fortement éliminé.

7. Procédé selon la revendication 1, **caractérisé en ce qu'**une seconde étape d'élimination de tendances (243) fournit le signal final D(s(t)) - D (s(t)) avec une fréquence dominante forte qui peut être utilisée directement dans le domaine de temps (260) et de fréquence (270).

8. Procédé selon la revendication 1, **caractérisé en ce que** la composante périodique du signal final est analysée en deux étapes différentes dans laquelle:
- L'analyse du domaine temporel (260) comprend les étapes consistant à:
• Application de la détection de crête (261) au signal temporel éliminé,
• l'utilisation des intervalles de crêtes estimés comme intervalles entre battements du signal de fréquence cardiaque
• Corrélation forte de la fréquence cardiaque et respiratoire pour limiter physiquement la variabilité temporelle des valeurs estimées
• l'utilisation d'une étape additionnelle d'enlèvement des aberrations (262) pour éliminer les fausses détections possibles,
et
- L'analyse du domaine de fréquence (270) comprend les étapes consistant à:
• Multiplier le signal de domaine de temps avec une fonction de fenêtre pour minimiser la fuite spectrale (271) sur la conversion du domaine de fréquence,
• Réaliser la transformation du domaine de fréquence (272) avec la méthode FFT à point N discret,
• Ignorer certains composants de fréquence en utilisant un filtre de fréquence passe bande idéal (273)
• Accepter la crête dominante du signal de fréquence dans le filtre passe-bande sélectionné (274) comme la fréquence du signe vital à l'étape finale.

9. Procédé selon la revendication 1, **caractérisé en ce que** les valeurs de couleur pour l'analyse du signe vital sont obtenues alternativement en utilisant le modèle d'apparence généré ainsi que les valeurs de couleur réelles de la région faciale d'intérêt.

10. Procédé selon la revendication 1, **caractérisé en ce que** l'étape de d'élimination de tendances élimine efficacement la composante de bruit du signal.

11. Procédé selon la revendication 1, **caractérisé en ce que** les variations entre les intervalles de battement de la fréquence cardiaque sont éstimées.

12. Procédé selon la revendication 1, **caractérisé en ce qu'**il comprend une définition de zone d'intérêt unique ou multiple pour commander l'étape d'acquisition de signal de sorte que les informations relatives à PPG sont préservées dans lesquelles les expressions faciales et les mouvements musculaires sont considérés.

13. Procédé selon la revendication 1, **caractérisé en ce que** les mouvements du muscle facial et de la tête sont compensés pour permettre un mouvement libre de la tête lorsque l'acquisition du signal est effectuée en utilisant le suivi du repère facial de l'apparence active.
